# EUROPEAN PATENT APPLICATION

(11) **EP 3 679 858 A1**
(43) Date of publication of application: **15.07.2020**
(21) Application number: 19860261.7
(22) Date of filing: 01.08.2019
(51) Int. Cl.: A61B 5/00

(54) **PHOTOPLETHYSMOGRAPHY DEVICE, AND TERMINAL**

(30) Priority: 14.09.2018 CN 201811076602; 14.09.2018 CN 201821514003 U
(71) Applicant: Anhui Huami Information Technology Co., Ltd., Hefei, Anhui 230088 (CN)
(72) Inventor: HOU, Shien Chang, Hefei, Anhui 230088 (CN); LI, Kai, Hefei, Anhui 230088 (CN)
(74) Representative: Papa, Elisabetta
(86) International application number: PCT/CN2019/098824
(87) International publication number: WO 2020/052363

(57) **Abstract**

The present disclosure provides a photoplethysmograph device and a terminal, belonging to the technical field of terminals. The photoplethysmograph device includes a housing provided with a mounting hole; an optical device accommodated in the mounting hole; and a light-transmitting member including a first end face, a side face connected to the first end face, and a second end face away from the first end face and connected to the side face. The light-transmitting member is connected to the housing and blocks the mounting hole, and the first end face is disposed near the optical device; the side face includes a reflecting face configured to totally reflect light incident on the reflecting face from the first end face to the second end face so as to emit the light, or totally reflect light incident on the reflecting face from the second end face to the first end face so as to emit the light.

## Description

### FIELD

The present disclosure relates to a technical field of terminals, and in particular, to a photoplethysmograph device and a terminal.

### BACKGROUND

A photoplethysmograph device is a detection device that acquires physiological signals by detecting photoplethysmographic signals of human bodies. Typically, the photoplethysmograph device includes a housing provided with a mounting hole, and an optical device accommodated in the mounting hole and configured to acquire photoplethysmographic signals. The optical device includes a light emitter and a light sensor. Moreover, in a common photoplethysmograph device, there is a shielding portion between the light sensor and the light emitter to prevent light from reaching the light sensor directly instead of entering the skin and hence from forming an interference signal.

However, due to the presence of the shielding portion and the housing, part of the light emitted from the light emitter cannot exit from the mounting hole due to a large angle, but is irradiated onto a side wall of the mounting hole and absorbed or blocked by the housing, resulting in a low efficiency and utilization rate of the light emitter. In addition, the angle of light reaching the skin through a window depends on the luminescent characteristics of the light emitter itself. A considerable part of the light travels away from the light sensor and cannot be received by the light sensor to form a signal; and another part of the light reaches the light sensor without going deep into a blood-rich portion of the skin, and thus forms an invalid signal.

### SUMMARY

The present disclosure provides a photoplethysmograph device and a terminal, to solve the defects in the related art.

A first aspect of the present disclosure provides a photoplethysmograph device. The photoplethysmograph device includes: a housing provided with a mounting hole; an optical device accommodated in the mounting hole; and a light-transmitting member including a first end face, a side face connected to the first end face, and a second end face away from the first end face and connected to the side face. The light-transmitting member is connected to the housing and blocks the mounting hole, and the first end face is disposed near the optical device. The side face includes a reflecting face configured to totally reflect light incident on the reflecting face from the first end face to the second end face so as to emit the light, or totally reflect light incident on the reflecting face from the second end face to the first end face so as to emit the light.

Optionally, the optical device includes: at least one light emitter configured to emit detection light; and at least one light sensor configured to receive detection light carrying detection information. The at least one light emitter and the at least one light sensor are accommodated in different mounting holes respectively.

Optionally, the reflecting face includes a curved face or a plurality of inclined faces connected with each other.

Optionally, the first end face includes a first condensing face protruding toward an inside of the mounting hole.

Optionally, the first end face further includes a recessed face recessed toward an outside of the mounting hole, and the recessed face is disposed to surround the first condensing face.

Optionally, the second end face includes a second condensing face protruding toward the outside of the mounting hole.

Optionally, the light-transmitting member includes a first light-transmitting member cooperating with the light emitter, and a second light-transmitting member cooperating with the light sensor. A curvature or gradient of a side of a reflecting face of the first light-transmitting member away from the second light-transmitting member is configured in such a way that the light totally reflected by the reflecting face is emitted in a direction biased towards the second light-transmitting member.

Optionally, a light-emitting axis of the light emitter is biased toward a side where the light sensor is located.

Optionally, the side face further includes a connecting face connected to an inner wall of the mounting hole.

Optionally, the photoplethysmograph device further includes a connecting member connected to the side face of the light-transmitting member and the inner wall of the mounting hole, and a refractive index of a material of the connecting member is lower than a refractive index of a material of the light-transmitting member.

Optionally, the connecting member and the housing are of an integrally formed structure.

Optionally, the photoplethysmograph device further includes a light-transmitting plate disposed outside the mounting hole and connected to the housing, and the light-transmitting plate is further connected with the second end face of the light-transmitting member.

A second aspect of the present disclosure provides a terminal including the above photoplethysmograph device provided by the first aspect.

It should be understood that the above general description and the following detailed description are intended to be exemplary and illustrative rather than restrictive.

The photoplethysmograph device and the terminal provided by the present disclosure at least have the following beneficial effects.

The photoplethysmograph device provided by the embodiments of the present disclosure totally reflects the light by means of the reflecting face of the light-transmitting member, so that the light originally projected on the inner wall of the mounting hole is totally reflected and emitted from the light-transmitting member, to avoid the light loss caused by projection on the side wall of the mounting hole. In such a case, when the optical device is a light emitter, more light can be emitted from the mounting hole by means of the light-transmitting member to form working light, which improves the efficiency and utilization rate of the light emitter and increases the working light intensity of the photoplethysmograph device; when the optical device is a light sensor, more light can enter the mounting hole through the light-transmitting member, so as to be received by the optical device, reducing the amount of invalid light and improving the quality of signals obtained by the light sensor.

### BRIEF DESCRIPTION OF THE DRAWINGS

The accompanying drawings, which are incorporated in and constitute a part of this specification, illustrate embodiments consistent with the present disclosure and, together with the description, serve to explain the principles of the present disclosure.
FIG. 1 is a schematic diagram of a photoplethysmograph device in the related art.
FIG. 2 is a schematic diagram of a photoplethysmograph device according to an exemplary embodiment.
FIG. 3 is a schematic diagram of a light-transmitting member according to an exemplary embodiment.
FIG. 4 is a schematic diagram of a light-transmitting member according to another exemplary embodiment.
FIG. 5 is a schematic diagram of a photoplethysmograph device according to another exemplary embodiment.
FIG. 6 is a schematic diagram of a light-transmitting member according to another exemplary embodiment.
FIG. 7 is a schematic diagram of a light-transmitting member according to another exemplary embodiment.
FIG. 8 is a schematic diagram of a photoplethysmograph device using the light-transmitting member illustrated in FIG. 7 according to an exemplary embodiment.
FIG. 9 is a schematic diagram of a photoplethysmograph device according to another exemplary embodiment.
FIG. 10 is a schematic diagram of a light-transmitting member according to another exemplary embodiment.
FIG. 11 is a schematic diagram of a photoplethysmograph device according to another exemplary embodiment.
FIG. 12 is a schematic diagram of a photoplethysmograph device according to another exemplary embodiment.
FIG. 13 is a schematic diagram of a photoplethysmograph device according to another exemplary embodiment.
FIG. 14 is a schematic diagram of a photoplethysmograph device according to another exemplary embodiment.
FIG. 15 is a schematic diagram of a photoplethysmograph device according to another exemplary embodiment.

### Reference numerals:

- 1: housing;
- 11: mounting hole;
- 111: top wall of mounting hole;
- 2: optical device;
- 2A: light emitter;
- 2B: light sensor;
- 3: light-transmitting member;
- 3A: first light-transmitting member;
- 3B: second light-transmitting member;
- 31: first end face;
- 311: first condensing face;
- 312: recessed face;
- 32: reflecting face;
- 321: inclined face;
- 33: second end face;
- 331: second condensing face;
- 34: connecting face;
- 4: connecting member;
- 5: light-transmitting plate.

### DETAILED DESCRIPTION

Exemplary embodiments of the present disclosure will be described in detail and examples of the embodiments will be illustrated in the drawings. When the following description refers to the drawings, unless specified otherwise, the same numbers in different drawings represent the same or similar elements. The implementations described in the following exemplary embodiments do not represent all implementations consistent with the present disclosure, and instead they are merely examples of devices and methods consistent with aspects of the present disclosure as detailed in the appended claims.

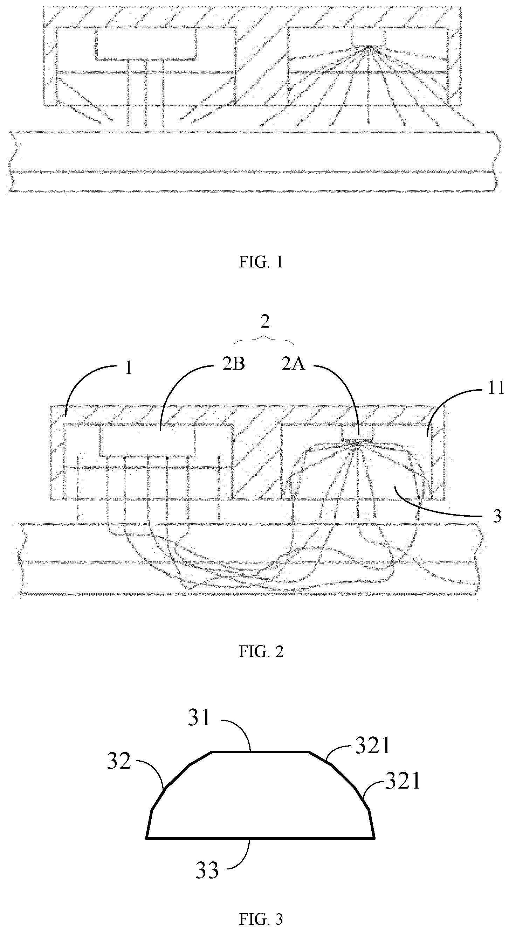

FIG. 1 is a schematic diagram of a photoplethysmograph device in the related art. As illustrated in FIG. 1, when light exits or enters the mounting hole, a part of large-angle emergent light or a part of large-angle incident light is projected on a side wall of the mounting hole, causing loss. Such a situation can impair the detection light intensity that can be emitted or received by the optical device, thereby affecting detection effect of the photoplethysmograph device.

FIG. 2 is a schematic diagram of a photoplethysmograph device according to an exemplary embodiment. As illustrated in FIG. 2, the photoplethysmograph device includes a housing 1, an optical device 2, and a light-transmitting member 3. The housing 1 is provided with a mounting hole 11; the optical device 2 is accommodated in the mounting hole 11; the light-transmitting member 3 includes a first end face 31, a side face connected to the first end face 31, and a second end face 33 away from the first end face 31 and connected to the side face 32. The light-transmitting member 3 is connected to the housing 1 and blocks the mounting hole 11, and the first end face 31 is disposed near the optical device 2. The side face includes a reflecting face 32, and the reflecting face 32 is configured to totally reflect light incident on the reflecting face 32 from the first end face 31 to the second end face 33 so as to emit the light, or totally reflect light incident on the reflecting face 32 from the second end face 33 to the first end face 31 so as to emit the light.

When the optical device 2 is a light emitter 2A configured to emit detection light, the light enters the light-transmitting member 3 via the first end face 31, and a part of the light directly exits the light-transmitting member 3 through the second end face 33, while a part of the light projected on the reflecting face 32 of the light-transmitting member 3 is totally reflected and then exits the light-transmitting member 3 through the second end face 33.

In such a case, the light flux emitted from the mounting hole 11 can be increased by the light-transmitting member 3 to reduce the light loss, and thus the detection light intensity of the photoplethysmograph device is improved, and the efficiency and utilization rate of the light emitter 2A is enhanced. Moreover, compared with the photoplethysmograph device in the related art, the light emitter 2A in the photoplethysmograph device provided by the embodiments of the present disclosure requires lower power consumption while achieving the same light emission intensity.

When the optical device 2 is a light sensor 2B configured to receive detection light carrying detection information, the light enters the light-transmitting member 3 via the second end face 33, and a part of the light directly exits the light-transmitting member 3 through the first end face 31, while a part of the light projected on the reflecting face 32 of the light-transmitting member 3 is totally reflected and then exits the light-transmitting member 3 through the first end face 31. The effective detection light that can be received by the light sensor 2B is increased by the light-transmitting member 3, the amount of invalid light is reduced, and the quality of signals acquired by the light sensor 2B is improved.

The photoplethysmograph device provided by the embodiment of the present disclosure totally reflects the light by means of the reflecting face 32 of the light-transmitting member 3, so that the light originally projected on an inner wall of the mounting hole 11 is totally reflected and then exits the light-transmitting member 3 through the first end face 31 (when the optical device 2 is the light sensor 2B) or through the second end face 33 (when the optical device 2 is the light emitter 2A). Thus, the light reflected out of the light-transmitting member 3 can be used as the detection light to enter a target detection area or be received by the light sensor to avoid loss.

In one embodiment, the optical device 2 includes at least one light emitter 2A and at least one light sensor 2B. Moreover, the light emitter 2A and the light sensor 2B are respectively accommodated in different mounting holes 11. The type and number of the optical devices 2 possessed by one photoplethysmograph device in the embodiments of the present disclosure is not specifically limited. For example, one photoplethysmograph device includes two light emitters 2A and one light sensor 2B, or includes two light sensors 2B and one light emitter 2A, etc.

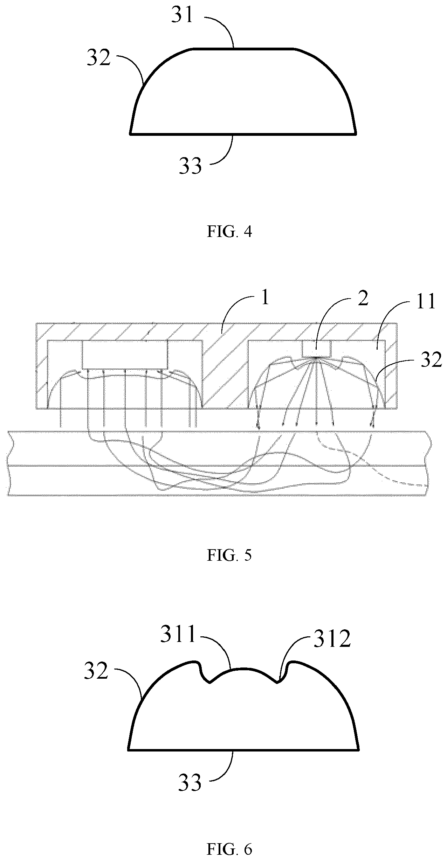

FIG. 3 is a schematic diagram of the light-transmitting member 3 according to an exemplary embodiment, and FIG. 4 is a schematic diagram of the light-transmitting member 3 according to another exemplary embodiment.

In one embodiment, with respect to the structure of the light-transmitting member 3, optionally, as illustrated in FIG. 3, the reflecting face 32 includes a plurality of inclined faces 321 connected with each another. The reflecting face 32 can totally reflect the incident light by adjusting gradients of the inclined faces 321. Alternatively, as illustrated in FIG. 4, the reflecting face 32 includes a curved face. The reflecting face 32 can totally reflect the incident light by adjusting the curvature of the curved face.

FIG. 5 is a schematic diagram of the photoplethysmograph device according to another exemplary embodiment; FIG. 6 is a schematic diagram of the light-transmitting member 3 according to another exemplary embodiment.

In one embodiment, as illustrated in FIGS. 5 and 6, the first end face 31 includes a first condensing face 311 that protrudes toward an inside of the mounting hole 11. A size of the first condensing face 311 can be selected according to a shape of the optical device 2. For example, when the optical device 2 has a working face (for example, a light source or a light receiving face) of a large size, the size of the first condensing face 311 may be appropriately increased to cover the working face of the optical device 2. The light converged by the first condensing face 311 and passing through the first end face 31 enhances directivity of the light and helps to increase the effective working light intensity.

Specifically, when the optical device 2 is the light emitter 2A, the light passing through the first end face 31 is the emergent light. The aggregation degree of the emergent light is improved by the first condensing face 311, so that as much emergent light as possible reaches the blood-rich skin layer to perform detection, which helps to increase the effective detection signals.

When the optical device 2 is the light sensor 2B, the light passing through the first end face 31 is the incident light, and the aggregation degree of the incident light is improved by the first condensing face 311, so that the incident light is projected on the light sensor 2B as much as possible to generate the effective detection signals, which helps to collect photoplethysmographic signals.

Further, still referring to FIG. 5 and FIG. 6, the first end face 31 further includes a recessed face 312 that is recessed toward an outside of the mounting hole 11, and the recessed face 312 is disposed to surround the first condensing face 311. By providing the recessed face 312 surrounding the first condensing face 311, the thickness of the light-transmitting member 3 can be reduced, thereby reducing the depth of the mounting hole 11 and the thickness of the overall photoplethysmograph device, to make the photoplethysmograph device smaller and lighter. In addition, the arrangement of the recessed face 312 helps the light-transmitting member 3 to enclose the optical device 2, such that the large-angle light emitted by the light emitter 2A reaches the reflecting face 32 of the light-transmitting member 3 or allows the light sensor 2B to fully receive the large-angle light reflected by the reflecting face 32.

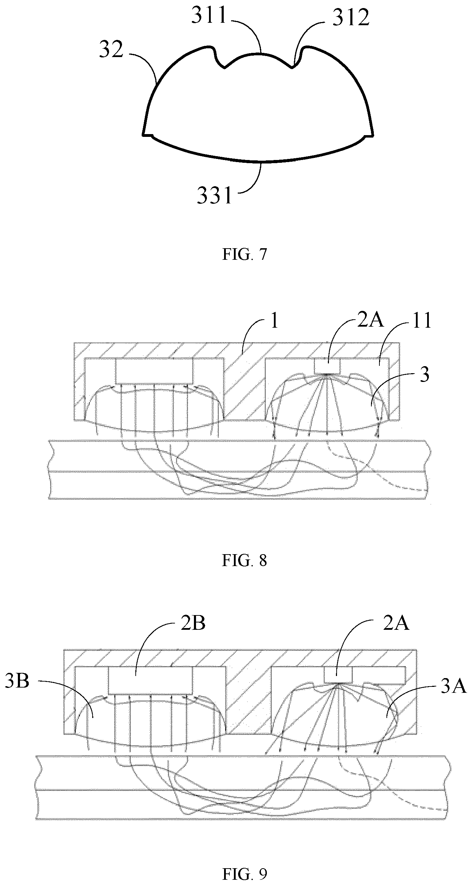

FIG. 7 is a schematic diagram of the light-transmitting member according to another exemplary embodiment, and FIG. 8 is a schematic diagram of the photoplethysmograph device using the light-transmitting member illustrated in FIG. 7 according to an exemplary embodiment.

In one embodiment, as illustrated in FIGS. 7 and 8, the second end face 33 of the light-transmitting member 3 includes a second condensing face 331 that protrudes toward the outside of the mounting hole 11. Similar to the first condensing face 311, the second condensing face 331 can assist in changing exit angle or incident angle of the light, enhance the aggregation degree of the light, and improve the light utilization efficiency.

Optionally, the first condensing face 311 and the second condensing face 331 are spherical or aspheric structures, and the aggregation effect of the light can be adjusted by the curvature regulation and control to assist in changing the direction of the light. Moreover, in use, the second end face 33 abuts against the skin of the person to be inspected, so that the second condensing face 331 optionally has a large curvature to avoid a foreign body sensation during use and optimize the user experience.

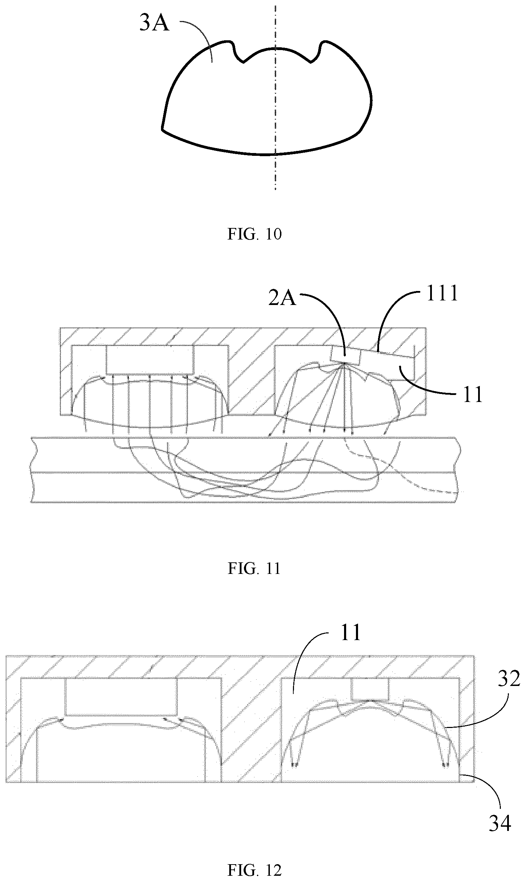

FIG. 9 is a schematic diagram of the photoplethysmograph device according to another exemplary embodiment; and FIG. 10 is a schematic diagram of the light-transmitting member in the photoplethysmograph device illustrated in FIG. 9.

In one embodiment, as illustrated in FIG. 9 and FIG. 10, the light-transmitting member 3 includes a first light-transmitting member 3A that cooperates with the light emitter 2A, and a second light-transmitting member 3B that cooperates with the light sensor 2B. The curvature or gradient of a side of a reflecting face 32 of the first light-transmitting member 3A away from the second light-transmitting member 3B is configured in such a way that the light totally reflected by the reflecting face 32 is emitted in a direction biased towards the second light-transmitting member 3B.

Optionally, the first light-transmitting member 3A is a non-axisymmetric light-transmitting member 3, and its side away from the second light-transmitting member 3B has a larger curvature or gradient, so that the light is emitted from the mounting hole 11 in a direction biased toward the light sensor 2B. The light is emitted in the direction biased toward the light sensor 2B, so that the light sensor 2B detects more light reflected by the skin, acquires more effective detection signals, and improves the light utilization efficiency of the photoplethysmograph device.

Further, in this embodiment, optionally, the mounting hole 11 which is adapted to the first light-transmitting member 3A has a non-axisymmetric structure to be fitted with the side face of the first light-transmitting member 3A.

Further, FIG. 11 is a schematic diagram of the photoplethysmograph device according to another exemplary embodiment. In one embodiment, as illustrated in FIG. 11, a light-emitting axis of the light emitter 2A is biased toward a side where the light sensor 2B is located. Optionally, the light emitter 2A is mounted on a flat and straight top wall 111 of the mounting hole in such a manner that the light-emitting axis is inclined; alternatively, the top wall 111 is an inclined face such that the light emitter 2A has a skewed light-emitting axis. The fact that the light-emitting axis of the light emitter 2A is biased toward the light sensor 2B further assists the light sensor 2B to obtain more effective detection signals.

In this embodiment, by adjusting the gradient of the optical axis of the light emitter 2A, the emergent light is emitted in a desired direction, thereby regulating and controlling an optical path of the light in the skin to enhance the quality of signals detected by the light sensor 2B. In such a case, it is possible to avoid the problems that the light is emitted in a direction away from the light sensor 2B and cannot reach the light sensor 2B to form the detection signal in the related art.

FIG. 12 is a schematic diagram of the photoplethysmograph device according to another exemplary embodiment. Regarding the connection manner of the housing 1 and the light-transmitting member 3, in one embodiment, as illustrated in FIG. 12, the side face of the light-transmitting member 3 further includes a connecting face 34 connected to the inner wall of the mounting hole 11. Optionally, the connecting face 34 is connected to the reflecting face 32 and is disposed at an opening of the mounting hole 11. In this embodiment, a contact area between the light-transmitting member 3 and the housing 1 is increased by the connecting face 34, thereby improving the connection stability.

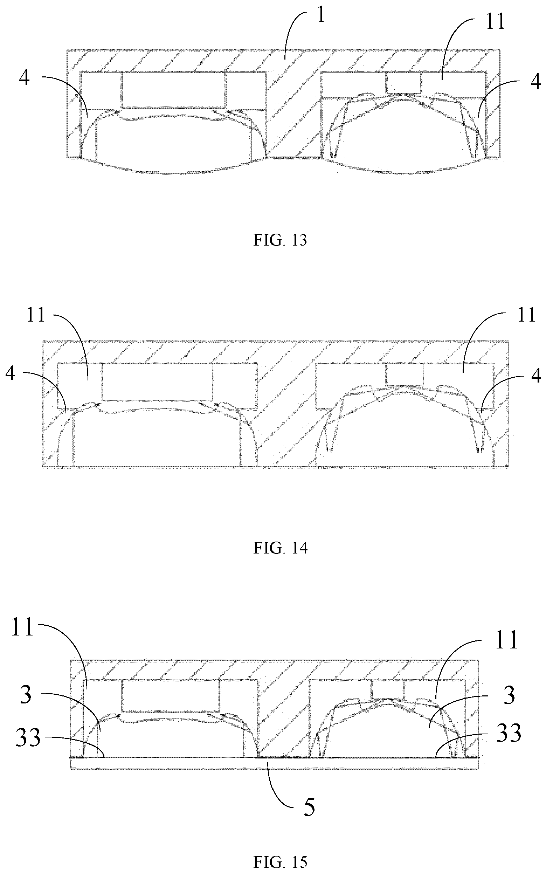

FIG. 13 is a schematic diagram of the photoplethysmograph device according to another exemplary embodiment, and FIG. 14 is a schematic diagram of the photoplethysmograph device according to another exemplary embodiment.

Regarding the connection manner of the housing 1 and the light-transmitting member 3, in another embodiment, as illustrated in FIG. 13, the photoplethysmograph device further includes a connecting member 4 connected to the side face of the light-transmitting member 3 and the inner wall of the mounting hole 11. The refractive index of the material of the connecting member 4 is lower than the refractive index of the material of the light-transmitting member 3.

It could be understood that when the reflecting face 32 of the light-transmitting member 3 is a curved face or includes a plurality of inclined faces connected with each other, an area of the junction between the side face and the inner wall of the mounting hole 11 is small, and the connection stability is weak. Therefore, in order to optimize the connection stability of the light-transmitting member 3 and the housing 1, the connecting member 4 is provided between the inner wall of the mounting hole 11 and the light-transmitting member 3. Moreover, the refractive index of the material of the connecting member 4 is lower than the refractive index of the material of the light-transmitting member 3, so as to achieve a purpose of totally reflecting the light by the reflecting face 32.

Optionally, the connecting member 4 is of a wedge-shaped structure, and has two faces connected to the inner wall of the mounting hole 11 and the side face of the light-transmitting member 3 respectively. The connecting member 4 has large contact areas with the mounting hole 11 and the light-transmitting member 3, which contributes to enhancement of the connection stability.

Optionally, as illustrated in FIG. 14, the connecting member 4 and the housing 1 are of an integrally formed structure to ensure the stable connection between the connecting member 4 and the housing 1, and help simplify the processing. However, in this optional embodiment, in terms of selecting the materials of the connecting member 4 and the housing 1, it should be noted that the housing 1 needs to achieve a shielding effect on the light emitter 2A and the light sensor 2B, and prevent the light emitted by the light emitter 2A from being received by the light sensor 2B without passing through the skin, which may otherwise cause interference.

In this embodiment, the stable connection between the housing 1 and the light-transmitting member 3 is achieved by the connecting member 4. In such a case, the side face of the light-transmitting member 3 can include only the reflecting face 32 to achieve sufficient reflection of light of different incident angles.

FIG. 15 is a schematic diagram of the photoplethysmograph device according to another exemplary embodiment. Regarding the connection manner of the housing 1 and the light-transmitting member 3, in another embodiment, as illustrated in FIG. 15, the photoplethysmograph device further includes a light-transmitting plate 5, and the light-transmitting plate 5 is connected to an outside portion of the housing 1 surrounding the mounting hole 11 and is also connected to the second end face 33 of the light-transmitting member 3.

Optionally, when the light-transmitting plate 5 is connected to the second end face 33 of the light-transmitting member 3, the contact area between the light-transmitting plate 5 and the light-transmitting member 3 is increased; and since the light-transmitting plate 5 is connected to the outside portion of the housing 1 surrounding the mounting hole 11, the contact area between the light-transmitting plate 5 and the housing 1 is increased, thereby achieving the stable connection between the housing 1 and the light-transmitting member 3.

Optionally, when the light-transmitting plate 5 is in contact with the second end face 33 of the light-transmitting member 3, the light-transmitting member 3 is optionally in interference fit with the mounting hole 11, and the light-transmitting plate 5 functions as a protective casing and is connected to the housing 1 and located outside the mounting hole 11, to prevent the light-transmitting member 3 from coming out of the mounting hole 11.

A second aspect of embodiments of the present disclosure provides a terminal including the photoplethysmograph device provided by the first aspect. The terminal may be a smart wearable device, such as a bracelet or a foot ring, a medical detection device, or the like, which is not specifically limited in the embodiments of the present disclosure.

Other embodiments of the present disclosure will be apparent to those skilled in the art from consideration of the specification and practice of the present disclosure disclosed herein. The present disclosure is intended to cover any variations, uses, or adaptations of the present disclosure following the general principles of the present disclosure and include common knowledge or conventional technical means in the art which are not disclosed in the present disclosure. The specification and embodiments should be regarded as illustrative only, and the true scope and spirit of the present disclosure is indicated by the following claims.

## Claims

1. A photoplethysmograph device, comprising:
a housing provided with a mounting hole;
an optical device accommodated in the mounting hole; and
a light-transmitting member comprising a first end face, a side face connected to the first end face, and a second end face away from the first end face and connected to the side face;
wherein the light-transmitting member is connected to the housing and blocks the mounting hole, and the first end face is disposed near the optical device;
the side face comprises a reflecting face configured to totally reflect light incident on the reflecting face from the first end face to the second end face so as to emit the light, or totally reflect light incident on the reflecting face from the second end face to the first end face so as to emit the light.

2. The photoplethysmograph device according to claim 1, wherein the optical device comprises:
at least one light emitter configured to emit detection light; and
at least one light sensor configured to receive detection light carrying detection information;
wherein the at least one light emitter and the at least one light sensor are accommodated in different mounting holes respectively.

3. The photoplethysmograph device according to claim 1, wherein the reflecting face comprises a curved face or a plurality of inclined faces connected with each other.

4. The photoplethysmograph device according to claim 1, wherein the first end face comprises a first condensing face protruding toward an inside of the mounting hole.

5. The photoplethysmograph device according to claim 1 or 4, wherein the first end face further comprises a recessed face recessed toward an outside of the mounting hole, and the recessed face is disposed to surround the first condensing face.

6. The photoplethysmograph device according to claim 1, wherein the second end face comprises a second condensing face protruding toward an outside of the mounting hole.

7. The photoplethysmograph device according to claim 1 or 2, wherein the light-transmitting member comprises a first light-transmitting member cooperating with a light emitter, and a second light-transmitting member cooperating with a light sensor;
a curvature or gradient of a side of a reflecting face of the first light-transmitting member away from the second light-transmitting member is configured in such a way that the light totally reflected by the reflecting face is emitted in a direction biased towards the second light-transmitting member.

8. The photoplethysmograph device according to claim 7, wherein a light-emitting axis of the light emitter is biased toward a side where the light sensor is located.

9. The photoplethysmograph device according to claim 1, wherein the side face further comprises a connecting face connected to an inner wall of the mounting hole.

10. The photoplethysmograph device according to claim 1, further comprising a connecting member connected to the side face of the light-transmitting member and an inner wall of the mounting hole, a refractive index of a material of the connecting member being lower than a refractive index of a material of the light-transmitting member.

11. The photoplethysmograph device according to claim 1 or 10, wherein the connecting member and the housing are of an integrally formed structure.

12. The photoplethysmograph device according to claim 1, further comprising a light-transmitting plate disposed outside the mounting hole and connected to the housing, the light-transmitting plate being further connected with the second end face of the light-transmitting member.

13. A terminal, comprising a photoplethysmograph device according to any one of claims 1 to 12.
